# EUROPEAN PATENT APPLICATION

(11) **EP 3 981 401 A1**
(43) Date of publication of application: **13.04.2022**
(21) Application number: 20200382.8
(22) Date of filing: 06.10.2020
(51) Int. Cl.: A61K 31/4422, A61K 31/277, A61K 38/26, A61K 45/06, A61P 3/10

(54) **PHARMACEUTICAL COMBINATION FOR THE TREATMENT AND/OR PREVENTION OF DIABETES COMPRISING A CALCIUM CHANNEL BLOCKING AGENT AND AN INCRETIN MIMETIC**

(71) Applicant: InSphero AG, 8952 Schlieren (CH)
(72) Inventor: MIR-COLL, Joan, 8046 Zurich (CH); YESILDAG, Burcak, 8702 Zollikon (CH)
(74) Representative: Michalski Hüttermann & Partner Patentanwälte mbB

(57) **Abstract**

The present invention relates to pharmaceutical combination for the treatment and/or prevention of diabetes, comprising a calcium channel blocking agent an an incretin.

## Description

This invention relates to field of treating and/or preventing diabetes and especially in novel uses of compounds and /or treatment and or prevention methods for diabetes.

Diabetes mellitus is a chronic disease characterized by high blood glucose due to inadequate insulin production and/or insulin resistance. It affects around 400 million people worldwide and causes an estimated 5 million deaths per year.

Diabetes is generally divided into Type I and Type II-diabetes.

Type I diabetes (T1D) is a chronic condition in which the pancreas produces little or no insulin most commonly due to autoimmune destruction of the insulin-secreting β-cells within the pancreatic islets.

Type II diabetes (T2D) is caused by the stress-induced loss of functional β-cell mass due to the constant high demand to produce and secrete insulin caused by persistent hyperglycemia (cf. Zhang, 2019)

T2D is steadily increasing and represents a worldwide health problem. The majority of cases occur secondary to obesity with its associated insulin resistance. T2D develops after years of prediabetes, or impaired glucose tolerance (IGT). The disease manifests when insulin resistance is no longer compensated by augmented insulin secretion (Aroda et al., 2017; Ligthart et al., 2016; Weir and Bonner-Weir, 2004).

Accordingly, monitoring the insulin secretory capacity of pancreatic β-cells best predicts future diabetes (Lyssenko et al., 2008). During prediabetes, blood glucose values gradually increase, exerting harmful effects on a wide variety of organs, including the cardiovascular system and the pancreatic β-cells, so-called glucotoxicity (Wajchenberg, 2007; Weir and Bonner-Weir, 2004).

Diabetes can be diagnosed in several ways as e.g. referred to on the American Diabetes Website (https://www.diabetes.org/alc/diagnosis). Typically, diabetes is diagnosed using one or more of the following:
- A1C test that measures average blood sugar for the past 2 to 3 months. Diabetes is diagnosed at an A1C of greater than or equal to 6.5%;
- Fasting Plasma Glucose (FPG) test that measures fasting blood glucose levels. Diabetes is diagnosed at fasting blood sugar of greater than or equal to 126 mg/dl;
- Oral Glucose Tolerance Test (OGTT) that measures blood sugar levels before and 2-hours after ingestion of a special sweet drink. Diabetes is diagnosed at 2-hour blood sugar of greater than or equal to 200 mg/dl.

In general, prediabetes is diagnosed using one or more of the following:
- A1C test that measures average blood sugar for the past 2 to 3 months. Diabetes is diagnosed at an A1C of 5.7% to 6.4%;
- Fasting Plasma Glucose (FPG) test that measures fasting blood glucose levels. Diabetes is diagnosed at fasting blood sugar of 100 to 125 mg/dl;
- Oral Glucose Tolerance Test (OGTT) that measures blood sugar levels before and 2-hours after ingestion of a special sweet drink. Diabetes is diagnosed at 2-hour blood sugar of 140 to 199 mg/dl.

In the present invention, prevention of diabetes includes treatment of prediabetes

Lifestyle modifications can prevent the outbreak of T2D and revert early stages of the disease but are difficult to implement. No current medication can efficiently preserve β-cell function or prevent T2D (Aroda et al., 2017). Among the commonly used T2D drugs, metformin acts mainly, but not exclusively, by suppressing glucose production in the liver (reviewed in Foretz et al., 2014), while thiazolidinediones enhance peripheral insulin sensitivity and sodium-glucose cotransporter-2 inhibitors lower blood glucose by preventing renal glucose reabsorption (Palanisamyet al., 2018).

Dysfunction of the β-cells with impaired glucose-stimulated insulin secretion (GSIS) is the main defect in T2D, since the reduction in β-cell mass varies greatly between different studies (cf. Marselli et al., 2014; Meier and Bonadonna, 2013). Additional antidiabetic drugs have therefore been introduced with the aim of increasing the GSIS in patients. Among these, sulfonylureas increase insulin secretion in a glucose-independent manner which may further contribute to exhaustion of already stressed β-cells, increase patients' risk for hypoglycemia and cause further weight gain (Wajchenberg, 2007). An additional class of antidiabetic medication that aims to improve GSIS in patents targets the pancreatic β-cells' incretin response.

Gastric inhibitory polypeptide (GIP; now referred to as glucose-dependent insulinotropic polypeptide) and GLP-1 are two endogenous incretins that are released from the gastrointestinal tract in response to nutrient ingestion to enhance GSIS from pancreatic β-cells (Yabe and Seino, 2011). After they are secreted, GLP-1 and GIP are rapidly degraded by the ubiquitous enzyme dipeptidyl peptidase-4 (DPP4), which inactivates incretins by cleaving their 2 N-terminal residues (Chon and Gautier, 2016). The effects of GLP-1 and GIP on healthy pancreatic β-cells are similar and additive. They together account for 50% to 70% of postprandial insulin secretion from pancreatic β-cells, known as the incretin effect (Drucker DJ, 2006). In addition to their insulinotropic effects, incretin hormones increase insulin gene expression, pancreatic β-cell survival and proliferation

The incretin effect is substantially decreased or no longer present in patients with T2D (Dogruel, and Balci, 2019). The secretion of GIP and GLP-1 appears to be relatively unchanged in type 2 diabetic patients (Meier and Nauck, 2010). The loss of incretin effect is the consequence of a diminished potency of GIP and decreased action of the already less potent GLP-1 on pancreatic β-cells (Meier and Nauck, 2010). GLP-1 mimetics or enhancers (DPP4 -inhibitors) are nevertheless used as antidiabetic treatment as they are able to potentiate insulin secretion and reduce plasma glucose concentrations at supraphysiological concentrations (Kjems, 2003)..

It is therefore an object to provide uses and/or methods for treating and/or preventing diabetes, especially to provide novel uses and/or treatments for increasing or re-potentiating of incretin response of pancreatic β-cell in T2D patients.

This object is solved by Claim 1 of the present invention. Accordingly, a pharmaceutical combination for the treatment and/or prevention of diabetes is provided, comprising
a) A calcium channel blocking agent or a pharmaceutically acceptable salt thereof;
b) An incretin mimetic and/or an incretin enhancer or a pharmaceutically acceptable salt thereof.

Surprisingly, a synergistic effect between both agents, leading to an increase in GSIS, could be observed throughout a wide range of applications within the present invention.

This was insofar unforseeable as actually it is believed that in T2D-patients incretins are less active, (cf. e.g Nauck and Meier, 2018).

Additionally, a method of treating and/or preventing diabetes in a patient is provided, comprising the steps of administering a combination of
a) A calcium channel blocking agent or a pharmaceutically acceptable salt thereof;
b) An incretin mimetic and/or an incretin enhancer or a pharmaceutically acceptable salt thereof,
to a patient in an amount sufficiently to observe an increase in glucose-stimulated insulin secretion (GSIS).

Additionally, a pharmaceutical combination for the treatment and/or prevention of T2D is provided, comprising
a) A calcium channel blocking agent or a pharmaceutically acceptable salt thereof;
b) An incretin mimetic and/or an incretin enhancer or a pharmaceutically acceptable salt thereof.

Additionally, a method of treating and/or preventing T2D in a patient is provided, comprising the steps of administering a combination of
a) A calcium channel blocking agent or a pharmaceutically acceptable salt thereof;
b) An incretin mimetic and/or an incretin enhancer or a pharmaceutically acceptable salt thereof
to a patient in an amount sufficiently to observe an increase in glucose-stimulated insulin secretion (GSIS).

Additionally, the present invention relates to a pharmaceutical combination for increasing glucose-stimulated insulin secretion (GSIS) in a patient, comprising
a) A calcium channel blocking agent or a pharmaceutically acceptable salt thereof;
b) An incretin mimetic and/or an incretin enhancer or a pharmaceutically acceptable salt thereof.

Additionally, a method of increasing the glucose-stimulated insulin secretion (GSIS) in a patient is provided, comprising the steps of administering a combination of
a) A calcium channel blocking agent or a pharmaceutically acceptable salt thereof;
b) An incretin mimetic and/or an incretin enhancer or a pharmaceutically acceptable salt thereof
to a patient in an amount sufficiently to observe an increase in glucose-stimulated insulin secretion (GSIS).

Additionally, a pharmaceutical combination for the treatment and/or prevention of Type II diabetes (T2D) in patients suffering from prehypertension and/or hypertension is provided, comprising
a) A calcium channel blocking agent or a pharmaceutically acceptable salt thereof;
b) An incretin mimetic and/or an incretin enhancer or a pharmaceutically acceptable salt thereof.

Additionally, a method of treating and/or preventing Type II diabetes (T2D) in a patient suffering from prehypertension and/or hypertension is provided, comprising the steps of administering a combination of
a) A calcium channel blocking agent or a pharmaceutically acceptable salt thereof;
b) An incretin mimetic and/or an incretin enhancer or a pharmaceutically acceptable salt thereof
to a patient in an amount sufficiently to observe an increase in glucose-stimulated insulin secretion (GSIS).

Alternatively, a pharmaceutical combination for the treatment and/or prevention of Type II diabetes (T2D) in patients not suffering from prehypertension and/or hypertension is provided, comprising
a) A calcium channel blocking agent or a pharmaceutically acceptable salt thereof;
b) An incretin mimetic and/or an incretin enhancer or a pharmaceutically acceptable salt thereof.

Alternatively, a method of treating and/or preventing Type II diabetes (T2D) in a patient not suffering from prehypertension and/or hypertension is provided, comprising the steps of administering a combination of
a) A calcium channel blocking agent or a pharmaceutically acceptable salt thereof;
b) An incretin mimetic and/or an incretin enhancer or a pharmaceutically acceptable salt thereof
to a patient in an amount sufficiently to observe an increase in glucose-stimulated insulin secretion (GSIS).

The present invention furthermore relates to a pharmaceutical composition comprising the pharmaceutical combination.

The present invention furthermore relates to pharmaceutical composition for the treatment and/or prevention of diabetes is provided, comprising
a) A calcium channel blocking agent or a pharmaceutically acceptable salt thereof;
b) An incretin mimetic and/or an incretin enhancer or a pharmaceutically acceptable salt thereof.

Additionally, a method of treating and/or preventing diabetes in a patient is provided, comprising the steps of administering a pharmaceutical composition comprising
a) A calcium channel blocking agent or a pharmaceutically acceptable salt thereof;
b) An incretin mimetic and/or an incretin enhancer or a pharmaceutically acceptable salt thereof
to a patient in an amount sufficiently to observe an increase in glucose-stimulated insulin secretion (GSIS).

Additionally, a pharmaceutical composition for the treatment and/or prevention of Type II diabetes (T2D) is provided, comprising
a) A calcium channel blocking agent or a pharmaceutically acceptable salt thereof;
b) An incretin mimetic and/or an incretin enhancer or a pharmaceutically acceptable salt thereof.

Additionally, a method of treating and/or preventing Type II diabetes (T2D) in a patient is provided, comprising the steps of administering a pharmaceutical composition comprising
a) A calcium channel blocking agent or a pharmaceutically acceptable salt thereof;
b) An incretin mimetic and/or an incretin enhancer or a pharmaceutically acceptable salt thereof
to a patient in an amount sufficiently to observe an increase in glucose-stimulated insulin secretion (GSIS).

Additionally, the present invention relates to a pharmaceutical composition for increasing glucose-stimulated insulin secretion (GSIS) in a patient, comprising
a) A calcium channel blocking agent or a pharmaceutically acceptable salt thereof;
b) An incretin mimetic and/or an incretin enhancer or a pharmaceutically acceptable salt thereof.

Additionally, a method of increasing the glucose-stimulated insulin secretion (GSIS) in a patient is provided, comprising the steps of administering a pharmaceutical composition comprising
a) A calcium channel blocking agent or a pharmaceutically acceptable salt thereof;
b) An incretin mimetic and/or an incretin enhancer or a pharmaceutically acceptable salt thereof
to a patient in an amount sufficiently to observe an increase in glucose-stimulated insulin secretion (GSIS).

Additionally, a pharmaceutical composition for the treatment and/or prevention of Type II diabetes (T2D) in patients suffering from prehypertension and/or hypertension is provided, comprising
a) A calcium channel blocking agent or a pharmaceutically acceptable salt thereof;
b) An incretin mimetic and/or an incretin enhancer or a pharmaceutically acceptable salt thereof.

Additionally, a method of treating and/or preventing Type II diabetes (T2D) in a patient suffering from prehypertension and/or hypertension is provided, comprising the steps of administering a pharmaceutical composition comprising
a) A calcium channel blocking agent or a pharmaceutically acceptable salt thereof;
b) An incretin mimetic and/or an incretin enhancer or a pharmaceutically acceptable salt thereof
to a patient in an amount sufficiently to observe an increase in glucose-stimulated insulin secretion (GSIS).

Alternatively, a pharmaceutical composition for the treatment and/or prevention of Type II diabetes (T2D) in patients not suffering from prehypertension and/or hypertension is provided, comprising
a) A calcium channel blocking agent or a pharmaceutically acceptable salt thereof;
b) An incretin mimetic and/or an incretin enhancer or a pharmaceutically acceptable salt thereof.

Alternatively, a method of treating and/or preventing Type II diabetes (T2D) in a patient not suffering from prehypertension and/or hypertension is provided, comprising the steps of administering a pharmaceutical composition comprising
a) A calcium channel blocking agent or a pharmaceutically acceptable salt thereof;
b) An incretin mimetic and/or an incretin enhancer or a pharmaceutically acceptable salt thereof
to a patient in an amount sufficiently to observe an increase in glucose-stimulated insulin secretion (GSIS).

The present invention furthermore relates to the use of a pharmaceutical combination and/or a pharmaceutical composition according to the invention for increasing or re-potentiating of incretin response of pancreatic β-cell in T2D patients

Whenever throughout this application reference is made to a certain compound, all possible isomers, especially stereoisomers, may it be diastereomers, enantiomers, keto/enol-tautomers etc. are supposed to fall under that compound if in the art those are generelly referred to by the same name.

The term "effective amount" and /or "an amount sufficient(ly)" as used herein especially means and/or includes a dosage which is sufficient to be effective for the treatment of the patient compared with no treatment.

The term "pharmaceutical composition" as used herein especially means and/or includes a product comprising an active compound or a salt thereof together with pharmaceutical excipients such as buffer, preservative, and optionally a tonicity modifier and/or a stabilizer. Thus, a pharmaceutical composition is also known in the art as a pharmaceutical formulation.

The term "excipient" as used herein especially means and/or includes the chemical compounds which are normally added to pharmaceutical compositions, e.g. buffers, tonicity agents, preservatives and the like.

The term "treatment of a disease" as used herein especially means and/or includes the management and care of a patient having developed the disease, condition or disorder.

The purpose of treatment is to combat the disease, condition or disorder. Treatment includes the administration of the active compounds to eliminate or control the disease, condition or disorder as well as to alleviate the symptoms or complications associated with the disease, condition or disorder.

The term "pharmaceutically acceptable salt" as used herein especially means and/or includes a derivative of a compound, modified by making acid or base salts of the compound. For example, acid salts may prepared from the free base (typically wherein the neutral form of the compound has a neutral -NH2 group) involving reaction with a suitable acid. Suitable acids for preparing acid salts include both organic acids, e.g., acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methane sulfonic acid, ethane sulfonic acid, p-toluenesulfonic acid, salicylic acid, and the like, as well as inorganic acids, e.g., hydrochloric acid, hydrobromic acid, sulfuric acid, nitric acid phosphoric acid and the like.

Conversely, preparation of basic salts of acid moieties which may be present on a compound may be prepared using a pharmaceutically acceptable base such as sodium hydroxide, potassium hydroxide, ammonium hydroxide, calcium hydroxide, trimethylamine or the like.

With regard to that stated above, it can be seen that the expression "pharmaceutically usable salt", or also "pharmaceutically acceptable salt" in the present connection is especially to be taken to mean and/or include a compound according to the invention which is in the form of one of its salts, in particular if this salt form of the compound imparts improved pharmacokinetic properties compared with its free form. The pharmaceutically acceptable salt form of the compound may also provide this compound with a desired pharmacokinetic property for the first time and can even have a positive influence on the pharmacodynamics of the compound with respect to its therapeutic efficacy in the body.

The term "hypertension" and/or" prehypertension especially means and /or includes the following:
Hypertension can be diagnosed in several ways as e.g. referred to on the American Heart Association Website (https://www.heart.org/en/health-topics/high-blood-pressure). Typically, hypertension (also referred to as high blood pressure) is diagnosed as any of the following:
- High blood pressure (hypertension) stage 1: SYSTOLIC mm Hg of 130 - 139, or DIASTOLIC mm Hg of 80-89
- High blood pressure (hypertension) stage 2: SYSTOLIC mm Hg of 140 or higher, or DIASTOLIC mm Hg of 90 or higher

Prehypertension can be diagnosed e.g. as referred to on the American Heart Association Website (https://www.heart.org/en/health-topics/high-blood-pressure). Typically, prehypertension (also referred to as elevated blood pressure) is diagnosed as the following: SYSTOLIC mm Hg of 120 - 129 and DIASTOLIC mm Hg of less than 80.

The calcium channel blocking agent, the incretin mimetics and/or incretin enhancers will be described in more detail from now on whereby all preferred embodiments or applications of each can be combined *ad libitum.*

### a) Calcium channel blocking agent

The term "calcium channel blocking agent " as used herein especially means and/or includes a group of compounds that disrupt the movement of calcium (Ca²⁺) through L-, N-, P/Q, R-, and T-type voltage-dependent calcium channels.

According to a preferred embodiment the calcium channel blocking agent is selected out of the group comprising dihydropyridines, phenylalkylamines, benzothiazepines or mixtures thereof.

### I) Dihydropyridines

According to a preferred embodiment of the present invention, the calcium channel blocking agent can comprise a dihydropyridine compound. This term especially means and/or includes a 1,4- dihydropyridine of the following structure:

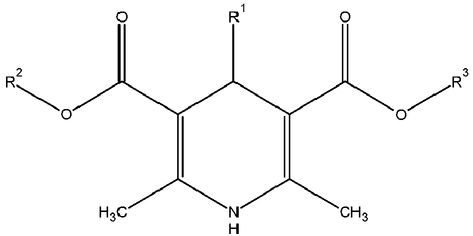

With R¹ being substituted or unsubstituted phenyl and R² and R³ independently from each other being substituted or unsubstituted C₁-C₆-alkyl.

Especially preferred 1,4-dihydropyridines are selected out of the group comprising amlodipine (e.g., sold under trade name Norvasc; e.g., Chemical Abstract Service Number (CAS) 88150-42-9), aranidipine (e.g., trade name Sapestra; e.g., CAS 86780-90-7), azelnidipine (e.g., trade name CalBlock or AZUSA; e.g., CAS 123524-52-7), barnidipine (e.g., alternative name mepirodipine; e.g., CAS 104713-75-9), benidipine (e.g., alternative names Benidipinum or Coniel; e.g., CAS 105979-17-7), cilnidipine (e.g., trade name Atelec or Cilacar; e.g., CAS 132203-70-4), clevidipine (e.g., trade name Cleviprex; e.g., CAS 167221-71-8), cronidipine (e.g., CAS 113759-50-5), darodipine (e.g., CAS 72803-02-2), dexniguldipine (e.g., CAS 120054-86-6), efonidipine (e.g., trade name Landel; e.g., CAS 111011-63-3), elgodipine (e.g., CAS 119413-55-7), elnadipine (e.g., CAS 103946-15-2), felodipine (e.g., trade name Plendil; e.g., CAS 72509-76-3), flordipine (e.g., CAS 77590-96-6), furnidipine (e.g., CAS 138661-03-7), iganidipine (e.g., CAS 119687-33-1), isradipine (e.g., trade name DynaCirc or Prescal; e.g., CAS 75695-93-1), lacidipine (e.g., trade name Lacipil or Motens; e.g., CAS 103890-78-4), lemildipine (e.g., CAS 94739-29-4), lercanidipine (e.g., trade name Zanidip; e.g., CAS 100427-26-7), levamlodipine (e.g., trade name Asomex, Eslo, Espin or EsCordi Cor; e.g., CAS 103129-82-4), levniguldipine, manidipine (e.g., trade name Manyper; e.g., CAS 89226-75-5 or 120092-68-4), nicardipine (e.g., trade name Cardene; e.g., CAS 55985-32-5), nifedipine (e.g., trade name Adalat or Procardia; e.g., CAS 21829-25-4), niguldipine (e.g., CAS 113165-32-5), niludipine (e.g., CAS 22609-73-0), nilvadipine (e.g., CAS 75530-68-6), nimodipine (e.g., trade name Nimotop or Nymalize; e.g., CAS 66085-59-4), nisoldipine (e.g., trade name Sular, Baymycard, Syscor; e.g., CAS 63675-72-9), nitrendipine (e.g., trade name Baypress; e.g., CAS 39562-70-4), olradipine (e.g., CAS 115972-78-6), oxodipine (e.g., CAS 90729-41-2), palonidipine (e.g., CAS 96515-73-0), pranidipine (e.g., CAS 99522-79-9), ryodipine (e.g., trade name Foridon or Ryosidine; e.g., CAS 71653-63-9), sagandipine (e.g., CAS 126294-30-2), sornidipine (e.g., CAS 95105-77-4), teludipine (e.g., CAS 108687-08-7), tiamdipine (e.g., CAS 110646-15-6), trombodipine (e.g., CAS 113658-85-8), vatanidipine (e.g., CAS 116308-55-5) or mixtures thereof.

### II) Phenylalkylamines

According to a preferred embodiment of the present invention, the calcium channel blocking agent can comprise a phenylalkylamine compound. This term especially means and/or includes a phenylalkylamine of the following structure: 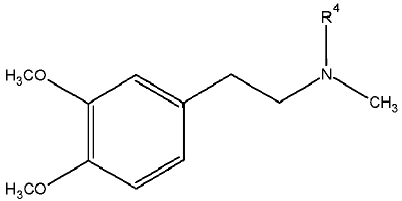 with R⁴ being substituted C₁-C₆-alkyl.

Especially preferred phenylalkylamines are selected out of the group comprising bepridil (e.g., trade name Vascor; e.g., CAS 64706-54-3), devapamil (e.g., CAS 92302-55-1), fendiline (e.g., CAS 13042-18-7), gallopamil (e.g., CAS 16662-47-8), verapamil (e.g., trade name Isoptin or Calan; e.g., CAS 52-53-9) or mixtures thereof.

### III) Benzothiazepines

According to a preferred embodiment of the present invention, the calcium channel blocking agent can comprise a benzothiazepine compound such as diltiazem (e.g., trade name Cardizem or Dilacorxr; e.g., CAS 42399-41-7).

### IV) Other voltage-gated calcium channel blockers

According to a preferred embodiment of the present invention, the calcium channel blocking agent can additionally or preferably comprise a mibefradil (e.g., trade name Posicor; e.g., CAS 116644-53-2), bepridil (e.g., trade name Vascor; e.g., CAS 64706-54-3), flunarizine (e.g., trade name Sibelium; e.g., CAS 52468-60-7), fluspirilene (e.g., trade name Redeptin; e.g., CAS 1841-19-6), gabapentin (e.g., trade name Neurontin; e.g., CAS 60142-96-3), pregabalin (e.g., trade name Lyrica; e.g., CAS 148553-50-8), and ziconotide (e.g., trade name Prialt; e.g., CAS 107452-89-1) or mixtures thereof.

According to a preferred embodiment of the present invention, the calcium channel blocking agent is selected from the group comprising amlodipine, aranidipine, azelnidipine, barnidipine, benidipine, cilnidipine, clevidipine, cronidipine, darodipine, dexniguldipine, efonidipine, elgodipine, elnadipine, felodipine, flordipine, furnidipine, iganidipine, isradipine, lacidipine, lemildipine, lercanidipine, levamlodipine, levniguldipine, manidipine, nicardipine, nifedipine, niguldipine, niludipine, nilvadipine, nimodipine, nisoldipine, nitrendipine, olradipine, oxodipine, palonidipine, pranidipine, ryodipine, sagandipine, sornidipine, teludipine, tiamdipine, trombodipine, bepridil, devapamil, fendiline, gallopamil, verapamil, diltiazem, mibefradil, bepridil, flunarizine, fluspirilene, gabapentin, pregabalin, and ziconotide or mixtures thereof.

According to a preferred embodiment of the present invention, the calcium channel blocking agent is selected from the group comprising nifedipine and/or verapamil.

### b) Incretin mimetics and/or incretin enhancers

The term "incretin mimetics and/or enhancers" as used herein especially means and/or includes the derivatives of metabolic gut hormones, gastric inhibitory polypeptide (GIP) and glucagon-like peptide-1 (GLP-1) and molecules, including small peptides and small molecules, that interact with and activate signal transduction through their receptors as well as molecules that enhance the stability and concentration of the naturally occurring incretins in the human body.

### I) Incretin Mimetics

According to a preferred embodiment of the present invention, the incretin mimetics is selected out of incretin analogues and other GLP-1 and GIP receptor agonists, especially the incretin mimetics are selected out of the group of compounds that copy, or mimic, the functions of the natural incretin hormones to help lower post-meal blood glucose levels by interacting with and activating signal transduction through GLP-1 and GIP receptors.

The term "analogue" as used herein referring to a polypeptide especially means and/or includes a modified peptide wherein one or more amino acid residues of the peptide have been substituted by other amino acid residues and/or wherein one or more amino acid residues have been deleted from the peptide and/or wherein one or more amino acid residues have been added to the peptide. Such addition or deletion of amino acid residues can take place at the N-terminal of the peptide and/or at the C-terminal of the peptide. Further, an analogue includes peptides wherein one or more amino acids have been modified, such as by covalent linkage along an amino acid side chain, such as by ester or ether groups, especially with esters of carboxylic acids having ten carbons or more, with palmitoyl especially preferred. Further, an analogue includes peptides that are capable of dual GLP-1 and GIP receptor agonism, such as Tirzepatide (also referred to as LY3298176, Frias J.P., et al, 2018).

Especially preferred are liraglutide (e.g., trade name Victoza; e.g., CAS 204656-20-2), semaglutide (e.g., trade name Ozempic or Rybelsus; e.g., CAS 910463-68-2), [D-Ala2]-GIP, exenatide (e.g., trade name Byetta or Bydureon; e.g., CAS 141758-74-9), lixisenatide (e.g., trade name Lyxumia or Adlyxin; e.g., CAS 320367-13-3), dulaglutide (e.g., trade name Trulicity; e.g., CAS 923950-08-7), albiglutide (e.g., trade name Eperzan or Tanzeum; e.g., CAS 782500-75-8) or mixtures thereof.

### II) Incretin enhancers

According to a preferred embodiment of the present invention, incretin enhancers are selected out of a group of compounds that preserve the incretin peptides naturally secreted in the human body by inhibiting its proteolytic degradation and inactivation through the action of DPP-4, thus leading to increased stimulation of insulin secretion and improved control of blood glucose concentrations.

Especially preferred are sitagliptin (e.g., trade name Januvia; e.g., CAS 486460-32-6), vildagliptin (e.g., trade name Galvus or Xiliarx or Jaira; e.g., CAS 274901-16-5), saxagliptin (e.g., trade name Onglyza; e.g., CAS 361442-04-8), linagliptin (e.g., trade name Tradjenta or Trajenta; e.g., CAS 668270-12-0), gemigliptin (e.g., trade name Zemiglo; e.g., CAS 911637-19-9), anagliptin (e.g., trade name Suiny; e.g., CAS 739366-20-2), teneligliptin (e.g., trade name Tenelia; e.g., CAS 760937-92-6), alogliptin (e.g., trade name Nesina or Vipidia; e.g., CAS 850649-62-6), trelagliptin (e.g., trade name Zafatek; e.g., CAS 865759-25-7), omarigliptin (e.g., alternative name MK-3102; e.g., CAS 1226781-44-7), evogliptin (e.g., trade name Suganon; e.g., CAS 1222102-29-5), gosogliptin (e.g., trade name SatRx; e.g., CAS 869490-23-3), dutogliptin (e.g., CAS 852329-66-9) or mixtures thereof.

According to a preferred embodiment of the invention the incretin mimetic and /or incretin enhancer is selected from liraglutide and/or semaglutide.

According to a preferred embodiment of the present invention the calcium channel blocking agent and the incretin may be linked to each other via non-covalent or covalent bonds.

Such linked agents are generally known or referred to in the art as codrugs. As per Das N., et al. (2010) a "Codrug or mutual prodrug is an approach where various effective drugs, which are associated with some drawbacks, can be modified by attaching with other drugs of same or different categories directly or via a linkage."

As per Aljuffali I., et al. (2016) the term codrug especially includes that a codrug "can be cleaved in the body to generate parent actives. The codrug itself can be inactive, less active, or more active than the parent agents. It has been demonstrated that codrugs possess some benefits over conventional drugs, including enhanced solubility, increased permeation for passing across biomembranes, prolonged half-life for extending the therapeutic period, and reduced toxicity." In the context of the present invention, the calcium channel blocking agent and the incretin mimetic and/or enhancer may be linked to each other via non-covalent or covalent bonds to form a codrug that is suitable to release an active form of the calcium channel blocking agent and the incretin in vivo. The skilled person is able to identify suitable means of linking the calcium channel blocking agent and the incretin mimetic and/or enhancer to release their active forms in vivo.

In another embodiment, the calcium channel blocking agent and the incretin mimetic and/or enhancer are provided in the form suitable for simultaneous dosage, including but not limited to the form of a single or more than one pill and/or a capsule, and/or in a form suitable for injection.

In another embodiment, the calcium channel blocking agent and the incretin mimetic and/or enhancer are provided in the form suitable for non-simultaneous dosage, such that the calcium channel blocking agent and the incretin mimetic and/or enhancer are provided separately, including but not limited to the form of a pill or a capsule, or in a form suitable for injection.

The introduction of the pharmaceutical composition and/or pharmaceutical combination into a cell or organism can be carried out in accordance with the invention in any manner which enables the pharmaceutical composition to be delivered into the blood of a patient as a consequence of which a glucose-stimulated insulin secretion (GSIS) is induced.

The pharmaceutical composition of the present invention can be administered orally, and/or by injection. The type of administration selected depends on the indication, the dose to be administered, individual-specific parameters, etc. In particular, the pharmaceutical composition of the present invention can be administered enterally (e.g. orally, rectally) or parenterally (e.g. intramuscularly, subcutaneously, intravenously, and intradermally, transdermally, transmucosally, transurethrally, vaginally, pulmonarily).
The type of administration selected depends on the indication, the dose to be administered, individual-specific parameters, etc. In particular, the various types of administration facilitate site-specific therapy, which minimizes side effects and reduces the active compound dose. Very particularly preferred injections are intradermal, subcutaneous, intramuscular or intravenous injection. The administration can be carried out, for example, with the aid of so-called vaccination guns or by means of syringes. It is also possible to provide the substance as an aerosol, which is inhaled by the organism, preferably a human patient.

A percutaneous application is also possible in various carrier media. There, various aiding techniques like iontophoresis can be applied. The application can for instance be made via hydrostatic pressure.

In one embodiment, the pharmaceutical composition of the present invention is applied orally. This also applies to suitable peptidic incretin mimetics and/or enhancers; indeed, whereas peptidic compounds have traditionally been applied subcutaneously or by intravenous application, recent approaches have been developed to render peptide-based therapeutics suitable for oral application. For example, semaglutide represents a recently developed peptide-based agent that essentially represents a liraglutide derivative modified to include a palmitoyl group. Whereas liraglutide is often applied subcutaneously, semaglutide is suitable for oral or subcutaneous application.

Additional details, characteristics and advantages of the object of the invention are disclosed in the subclaims and the following description of the respective figures --which in an exemplary fashion--show several inventive examples according to the invention. Such examples do not necessarily represent the full scope of the invention, however, and reference is made therefore to the claims and herein for interpreting the scope of the invention. It is to be understood that both the foregoing general description and the following detailed description are exemplary and explanatory only and are intended to provide further explanation of the present invention as claimed.
Fig. 1 shows the glucose-stimulated insulin secretion (GSIS) for four comparative examples (i.e. one control sample, one sample with GLTX medium, one with liraglutide alone and one with nifedipine alone) and one inventive example (liraglutide and nifedipine in combination).
Fig. 2 shows the glucose-stimulated insulin secretion (GSIS) for four comparative examples (i.e. one control sample, one sample with GLTX medium, one with semaglutide alone and one with nifedipine alone) and one inventive example (semaglutide and nifedipine in combination).
Fig. 3 shows the glucose-stimulated insulin secretion (GSIS) for four comparative examples (i.e. one control sample, one sample with GLTX medium, one with liraglutide alone and one with verapamil) and one inventive example (liraglutide and verapamil in combination).

General method for measuring the glucose-stimulated insulin secretion (GSIS):
3D InSight^{™} Islet Microtissues were produced by optimized dissociation and hanging-drop based scaffold free reaggregation of isolated primary human islet cells. This process allows for a precise control over the newly forming islet microtissue while ensuring homogeneous and native-like distribution of endocrine cells within each microtissue (Misun and Yesildag, 2020). The resulting uniform islets display long-term (>28 days) and robust pancreatic β-cell function enabling high-throughput and longitudinal study of pancreatic islet function, regeneration, and preservation (Misun and Yesildag, 2020). The expanded life span of 3D InSight^{™} Human Islet Microtissues additionally enables physiologically relevant disease modeling. The glucolipotoxicity (GLTX) induced islet injury assay makes use of the detrimental effects of prolonged exposure to high glucose and high free fatty acids on pancreatic islets to mimic T2D relevant β-cell dysfunction. The toxicity of this interventions can be assessed by measuring robust indicators of islet function (glucose stimulated insulin secretion, GSIS). 3D InSight^{™} Human Islet Microtissues were produced from isolated cadaveric islets that was donated by the next of kin with informed consent (MT-04-002-01, InSphero AG, Switzerland, lot hIsMT_151, UNOS ID# AHHY337, 42 years old, female, Caucasian, BMI 23.5, EBV and CMV IgG positive serology, HbAlc 5.4 %, death by stroke, no history of type 2 diabetes). The islet microtissues were cultured for 13 days (in a humidified incubator at 37°C in 5% CO2, 95% air) in either standard 3D InSight^{™} Human Islet Maintenance Medium (CS-07-005-01, InSphero AG, Switzerland) or 3D InSight^{™} Human Islet Glucolipotox Medium (CS-07-510-04 InSphero AG, Switzerland).

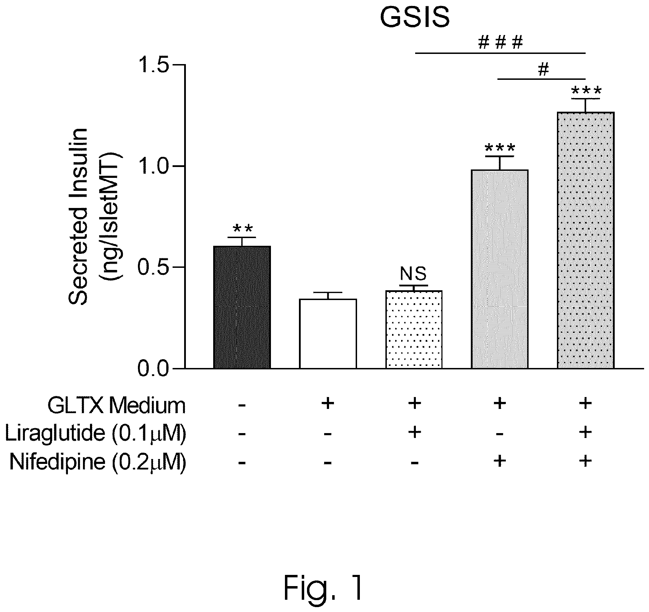

Human islet microtissues cultured in standard islet maintenance medium, which contains 5.5 mM glucose, were used as healthy controls. Human islet microtissues cultured in glucolipotox medium, which contains 11 mM glucose and an additional 300 µM of free fatty acids (200 µM of Oleic acid and 100 µM palmitic acid) were used as stressed controls. FFAs were bound to bovine serum albumin in 1:5 ratio, therefore matching BSA concentration was used for the healthy control groups, . The compound treatments were performed in glucolipotox medium for nifedipine (HY-B0284-1ML, Lucerna-Chem AG, Switzerland, at 0.2 µM), verapamil (HY-A0064-1ML, Lucerna-Chem AG, Switzerland, at 10 µM), semaglutide (NNC0113-0217, Novo Nordisk A/S, Denmark, at 1 µM) and liraglutide (NNC0090-1170, Novo Nordisk A/S, Denmark, at 0.1 or 1 µM), separately or combined, as illustrated in the description for Figs. 1 to 3 (see below).

Media and compounds were renewed every 2 or 3 days, with 70 µl of culture medium per well. DMSO was used as solvent for nifedipine and verapamil and its concentration was equalized in all the remaining wells. 6 technical replicates per group were used for all samples and each technical replicate represents one uniform islet microtissue cultured in an individual well of a Akura^{™} 96 well microplate (InSphero AG, Switzerland). As the starting material is homogenous for all data points, data normalization is not required.
After 13 days of incubation, conditioned culture media were removed and the glucose stimulated insulin secretion was performed in the absence of the compounds. Islet microtissues were washed twice with 70 µl of 3D InSight^{™} Krebs Ringer HEPES Buffer (KRHB, CS-07-051-01, InSphero AG, Switzerland) prepared following manufacturer's instructions and supplemented with 2.8 mM glucose. Next, 3D InSight^{™} Human Islet Microtissues were equilibrated in 70 µl of this solution for 1 hour. Following the equilibration, 3D InSight^{™} Human Islet Microtissues were washed twice with 70 µl of KRHB supplemented with 2.8 mM glucose and incubated with 55 µl of KRHB supplemented with 2.8 mM glucose for an additional 2 hours of static incubation. KRHB was removed and islet microtissues were washed twice with 70 µl of KRHB supplemented with 2.8 mM glucose. Next, 55 µl of KRHB supplemented with 16.7 mM glucose was added to each well to perform a glucose-stimulated insulin secretion (GSIS) assay. After 2 hours of static incubation, conditioned KRHB was collected for the analysis of stimulated insulin secretion. All the incubations were performed in a humidified incubator at 37°C in 5% CO2, 95% air. Insulin was quantified using Insulin Ultra-Sensitive HTRF Assay (62IN2PEH, Cisbio, France)..

Outliers were detected with ROUT outlier test (Q=5%). Statistical significance against the glucolipotox group (GLTX) was determined with One-way ANOVA and Dunnett's multiple comparisons test, rejecting the null hypothesis at p<0.05. "*", "**" and "***" represents p values smaller or equal to 0.05, 0.01 and 0.001 respectively. Statistical significance between the mono and combination therapies were determined with Student's t-test, rejecting the null hypothesis at p<0.05. "#", "# #" and "# # #" represents p values smaller or equal to 0.05, 0.01 and 0.001 respectively. "NS" represents not significant.

Fig. 1 shows the glucose-stimulated insulin secretion (GSIS) for four comparative examples (i.e. one control sample, one sample with GLTX medium, one with liraglutide alone and one with nifedipine alone) and one inventive example (liraglutide and nifedipine in combination).

As can be seen from Fig. 1, glucolipotox treatment results in decreased GSIS and long-term treatment with 0.1 µM liraglutide alone does not improve the GSIS. On the other hand, long-term treatment with 0.2 µM nifedipine alone has a positive influence on GSIS. However, GSIS is significantly higher for the inventive examples (combination of 0.1 µM liraglutide and 0.2 µM nifedipine) compared to the comparative examples containing either liraglutide or nifedipine alone. Most significantly, despite lacking long-term positive effects on insulin secretion, liraglutide in combination with nifedipine synergistically improves GSIS.

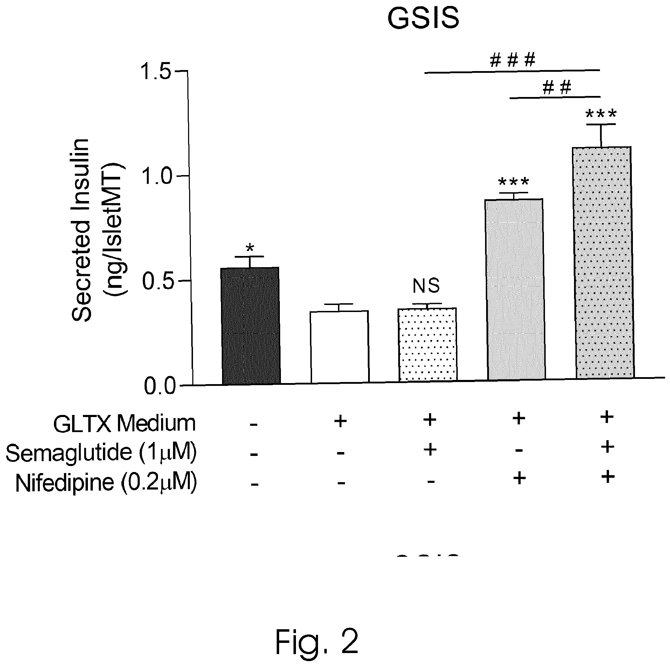

Fig. 2 shows the glucose-stimulated insulin secretion (GSIS) for four comparative examples (i.e. one control sample, one sample with GLTX medium, one with semaglutide alone and one with nifedipine alone) and one inventive example (semaglutide and nifedipine in combination).

As can be seen from Fig. 2, glucolipotox treatment results in decreased GSIS and long-term treatment with 1 µM semaglutide alone does not improve the GSIS. On the other hand, long-term treatment with 0.2 µM nifedipine alone has a positive influence on GSIS. However, GSIS is significantly higher for the inventive example (combination of 1 µM semaglutide and 0.2 µM nifedipine) compared to the comparative examples containing either semaglutide or nifedipine alone. Most significantly, despite lacking long-term positive effects on insulin secretion, semaglutide in combination with nifedipine synergistically improves GSIS.

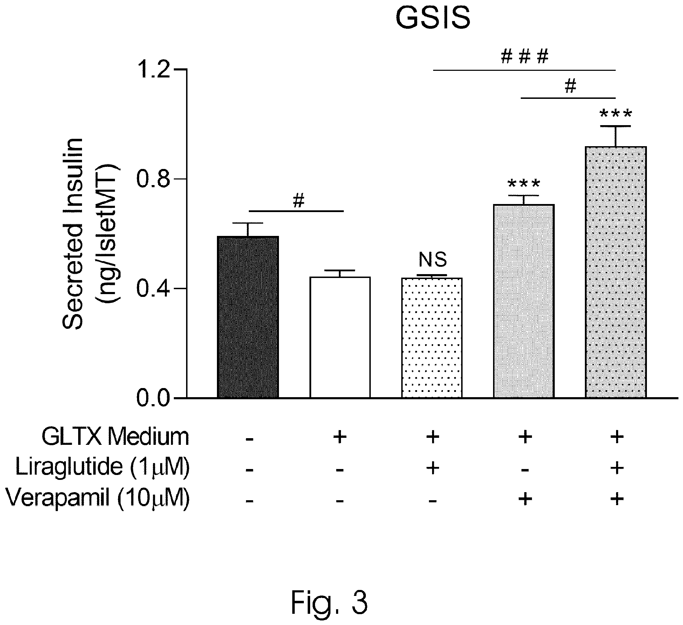

Fig. 3 shows the glucose-stimulated insulin secretion (GSIS) for four comparative examples (i.e. one control sample, one sample with GLTX medium, one with liraglutide alone and one with verapamil) and one inventive example (liraglutide and verapamil in combination).

As can be seen from Fig. 3, glucolipotox treatment results in decreased GSIS (significant only with t-test) and long-term treatment with 1 µM liraglutide alone does not improve the GSIS. On the other hand, long-term treatment with 10 µM verapamil alone has a positive influence on GSIS. However, GSIS is significantly higher for the inventive examples (combination of 1 µM liraglutide and 10 µM verapamil) compared to the comparative examples containing either liraglutide or verapamil alone. Most significantly, despite lacking long-term positive effects on insulin secretion, liraglutide in combination with verapamil synergistically improves GSIS.

The particular combinations of elements and features in the above detailed embodiments are exemplary only; the interchanging and substitution of these teachings with other teachings in this and the patents/applications incorporated by reference are also expressly contemplated. As those skilled in the art will recognize, variations, modifications, and other implementations of what is described herein can occur to those of ordinary skill in the art without departing from the spirit and the scope of the invention as claimed. Accordingly, the foregoing description is by way of example only and is not intended as limiting. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. The invention's scope is defined in the following claims and the equivalents thereto. Furthermore, reference signs used in the description and claims do not limit the scope of the invention as claimed.

### Literature:

Aljuffali, I.A., Lin, C.F. Chen, C.H. Fang, J.Y. (2016) The codrug approach for facilitating drug delivery and bioactivity, Expert Opinion on Drug Delivery, 2016, 1311-1325
Aroda, V.R., Knowler, W.C., Crandall, J.P., Perreault, L., Edelstein, S.L., Jeffries, S.L., Molitch, M.E., Pi-Sunyer, X., Darwin, C., Heckman-Stoddard, B.M., et al. (2017). Metformin for diabetes prevention: insights gained from the diabetes prevention program/diabetes prevention program outcomes study. Diabetologia 60, 1601-1611.
Chon, S., & Gautier, J. F. (2016). An Update on the Effect of Incretin-Based Therapies on β-Cell Function and Mass. Diabetes & metabolism journal, 40(2), 99-114.
Das, N. Dhanawat M., Dash, B. Nagarwal, R.C, Shrivastava, S. K. (2010) Codrug: An efficient approach for drug optimization, European Journal of Pharmaceutical Sciences Volume 41, Issue 5, 23 December 2010, Pages 571-588
Dogruel, H., & Balci, M. K. (2019). Development of therapeutic options on type 2 diabetes in years: Glucagon-like peptide-1 receptor agonist's role intreatment; from the past to future. World journal of diabetes, 10(8), 446-453.
Drucker D. J. (2006). The biology of incretin hormones. Cell metabolism, 3(3), 153-165.
Foretz, M., Guigas, B., Bertrand, L., Pollak, M., and Viollet, B. (2014). Metformin: from mechanisms of action to therapies. Cell Metabol. 20, 953-966.
Frias, J.P., et al. (2018) Efficacy and safety of LY3298176, a novel dual GIP and GLP-1 receptor agonist, in patients with type 2 diabetes: a randomized, placebo-controlled and active comparator-controlled phase 2 trial. The Lancet 392(10160), 2180-2193.
Kjems, L. L., Holst, J. J., Vølund, A., & Madsbad, S. (2003). The influence of GLP-1 on glucose-stimulated insulin secretion: effects on beta-cell sensitivity in type 2 and nondiabetic subjects. Diabetes, 52(2), 380-386. https://doi.org/10.2337/diabetes.52.2.380
Ligthart, S., van Herpt, T.T., Leening, M.J., Kavousi, M., Hofman, A., Stricker, B.H., van Hoek, M., Sijbrands, E.J., Franco, O.H., and Dehghan, A. (2016). Lifetime risk of developing impaired glucose metabolism and eventual pro-gression from prediabetes to type 2 diabetes: a prospective cohort study. Lancet Diabetes Endocrinol. 4, 44-51.
Marselli, L., Suleiman, M., Masini, M., Campani, D., Bugliani, M., Syed, F., Martino, L., Focosi, D., Scatena, F., Olimpico, F., et al. (2014). Are we overes-timating the loss of beta cells in type 2 diabetes? Diabetologia 57, 362-365.
Meier, J.J., and Bonadonna, R.C. (2013). Role of reduced beta-cell mass versus impaired beta-cell function in the pathogenesis of type 2 diabetes. Diabetes Care 36 (Suppl 2), S113-S119.
Meier, J. J., & Nauck, M. A. (2010). Is the diminished incretin effect in type 2 diabetes just an epi-phenomenon of impaired beta-cell function?. Diabetes, 59(5), 1117-1125.
Misun, P. M., Yesildag, B., Forschler, F., Neelakandhan, A., Rousset, N., Biernath, A., Hierlemann, A., & Frey, O. (2020). In Vitro Platform for Studying Human Insulin Release Dynamics of Single Pancreatic Islet Microtissues at High Resolution. Advanced biosystems, 4(3), e1900291
Nauck, M.A. & Meierk, J.J. (2018) Incretin hormones: Their role in health and disease, Diabetes Obes Metab. 2018 Feb;20 Suppl 1:5-21
Palanisamy, S., Yien, E.L.H., Shi, L.W., Si, L.Y., Qi, S.H., Ling, L.S.C., Lun, T.W., and Chen, Y.N. (2018). Systematic review of efficacy and safety of newer antidiabetic drugs approved from 2013 to 2017 in controlling HbAlc in diabetes patients. Pharmacy (Basel) 6
Wajchenberg, B.L. (2007). beta-Cell failure in diabetes and preservation by clinical treatment. Endocr. Rev. 28, 187-218.
Weir, G.C., and Bonner-Weir, S. (2004). Five stages of evolving beta-cell dysfunction during progression to diabetes. Diabetes 53 (Suppl 3), S16-S21.
Yabe, D., & Seino, Y. (2011). Two incretin hormones GLP-1 and GIP: comparison of their actions in insulin secretion and β cell preservation. Progress in biophysics and molecular biology, 107(2), 248-256.
Zhang, E., Al-Amily, I., Mohammed, S. Luan, C. Asplung, O. Ahmed M, Ye, Y. Ben-hail, D. Soni, A., Vishnu, N, Bompada, P., De Marinis, Y., Groopl, L., Shoshan-Barmatz, V, Renstro, E, Wollheim, C. and Salehi, A., (2019) Preserving Insulin Secretion in Diabetes by Inhibiting VDAC1 Overexpression and Surface Translocation in β-Cells, Cell Metabolism 29, 64-77

## Claims

1. A pharmaceutical combination for the treatment and/or prevention of diabetes, comprising:
a) A calcium channel blocking agent or a pharmaceutically acceptable salt thereof;
b) An incretin mimetic and/or an incretin enhancer or a pharmaceutically acceptable salt thereof.

2. A pharmaceutical combination of Claim 1 for the treatment and/or prevention of Type-II diabetes

3. A pharmaceutical combination of Claim 1 or 2 for the treatment and/or prevention of Type-II diabetes in patients not suffering from prehypertension and/or hypertension.

4. A pharmaceutical combination for increasing glucose-stimulated insulin secretion (GSIS) in a patient, comprising
a) A calcium channel blocking agent or a pharmaceutically acceptable salt thereof;
b) An incretin or a pharmaceutically acceptable salt thereof.

5. A pharmaceutical combination according to any of the claims 1 to 4, whereby the calcium-channel-blocking agent is selected from the group comprising dihydropyridines, phenylalkylamines, benzothiazepines or mixtures thereof.

6. A pharmaceutical combination according to any of the claims 1 to 5, whereby the calcium channel blocking agent is selected from the group comprising amlodipine, aranidipine, azelnidipine, barnidipine, benidipine, cilnidipine, clevidipine, cronidipine, darodipine, dexniguldipine, efonidipine, elgodipine, elnadipine, felodipine, flordipine, furnidipine, iganidipine, isradipine, lacidipine, lemildipine, lercanidipine, levamlodipine, levniguldipine, manidipine, nicardipine, nifedipine, niguldipine, niludipine, nilvadipine, nimodipine, nisoldipine, nitrendipine, olradipine, oxodipine, palonidipine, pranidipine, ryodipine, sagandipine, sornidipine, teludipine, tiamdipine, trombodipine, bepridil, devapamil, fendiline, gallopamil, verapamil, diltiazem, mibefradil, bepridil, flunarizine, fluspirilene, gabapentin, pregabalin, and ziconotide or mixtures thereof.

7. A pharmaceutical combination according to any of the claims 1 to 6 whereby the calcium channel blocking agent is nifedipine and/or verapamil.

8. A pharmaceutical combination according to any of the claims 1 to 7 whereby the incretin mimetic and/or an incretin enhancer is selected from liraglutide, semaglutide, [D-Ala2]-GIP, exenatide, lixisenatide, dulaglutide, albiglutide or mixtures thereof

9. A pharmaceutical combination according to any of the claims 1 to 8, whereby the incretin mimetic and/or an incretin enhancer is selected from sitagliptin, vildagliptin, saxagliptin, linagliptin, gemigliptin, anagliptin, teneligliptin, alogliptin, trelagliptin, omarigliptin, evogliptin, gosogliptin, dutogliptin or mixtures thereof.

10. A pharmaceutical combination according to any of the claims 1 to 9, whereby the incretin mimetic and/or an incretin enhancer is liraglutide and/or semaglutide.

11. A pharmaceutical composition comprising a pharmaceutical combination according to any of the claims 1 to 10.

12. The pharmaceutical composition according to Claim 11 for the treatment and/or prevention of diabetes

13. The pharmaceutical composition according to Claim 11 or 12 for the treatment and/or prevention of Type-II diabetes

14. The pharmaceutical composition according to any of the claims 11 to 13 for the treatment and/or prevention of Type-II diabetes in patients not suffering from prehypertension and/or hypertension

15. Use of a pharmaceutical combination according to any of the claims 1 to 10 and/or the pharmaceutical composition according to any of the claims Claim 11 to 14 for increasing or re-potentiating of incretin response of pancreatic β-cell in T2D patients
